# FASCICULE DE BREVET EUROPEEN

(11) **EP 0 683 897 B1**
(45) Date de publication et mention de la délivrance du brevet: **02.05.1997**
(21) Numéro de dépôt: 94906252.5
(22) Date de dépôt: 09.02.1994
(51) Int. Cl.: G01N 33/566, G01N 33/569, G01N 33/571, G01N 33/576

(54) **Procédé de détection de composés viraux au moyen d'une glycoprotéine**
Verfahren zum Nachweis von viralen Komponenten mittels eines Glykoproteins
Method for detecting viral components using a glycoprotein

(30) Priorité: 09.02.1993 FR 9301400
(43) Date de publication de la demande: 29.11.1995
(73) Titulaire: STEFAS, Elie, F-34280 La Grande-Motte (FR); RUCHETON, Marcel, F-34000 Montpellier (FR); GRAAFLAND, Hubert, F-34000 Montpellier (FR)
(72) Inventeur: STEFAS, Elie, F-34280 La Grande-Motte (FR); RUCHETON, Marcel, F-34000 Montpellier (FR); GRAAFLAND, Hubert, F-34000 Montpellier (FR)
(74) Mandataire: Peuscet, Jacques
(86) Numéro de dépôt international: FR9400142
(87) Numéro de publication internationale: WO9418569

(56) Documents cités:
- EP-A- 0 124 896

## Description

La présente invention concerne un procédé de détection et/ou de dosage de composés viraux et un support portant au moins une glycoprotéine.

Selon la présente invention, on entend par composé viral aussi bien les composés, en particulier protéiniques, constitutifs d'un virus, que des particules de type viral. Les particules de type viral sont, ou des virions, complets ou incomplets, ou des parties de virion, ou des assemblages contenant des composés constitutifs de virus, qui présentent certaines propriétés des virus ou des composés viraux ; en particulier, ils sont détectés par certains anticorps spécifiques de composés viraux ou pourraient être apparentés à ces composés viraux.

Selon la présente invention, on a trouvé que certains composés viraux se fixaient de façon spécifique sur une glycoprotéine : la β2'-glycoprotéine I ou une composition protéinique contenant la β2'-glycoprotéine I. La β2'-glycoprotéine I est la glycoprotéine décrite dans FR-A-2 701 263. La β2'-glycoprotéine I se fixe sur la membrane de particules virales, ainsi que sur certains composés ou surfaces chimiques ou biologiques. Selon la présente invention, on utilise cette propriété pour capter et isoler les composés viraux. ce qui permet de les détecter et/ou de les doser.

La présente invention a donc pour objet un procédé de détection et/ou de dosage de composés viraux, caractérisé par le fait qu'on fixe les composés viraux à l'aide de la β2'-glycoprotéine I.

Selon l'invention, la β2'-glycoprotéine I peut être utilisée pure ou sous forme de composition protéinique contenant en particulier d'autres glycoprotéines, éventuellement d'autres β2-glycoprotéines. Cette composition peut, en particulier, être celle obtenue par élution d'une colonne d'affinité sur un gel portant des groupes sulfates comme décrit dans FR-A-2 701 263.

Selon un premier mode de mise en oeuvre du procédé, on fixe la β2'-glycoprotéine I sur un support, on fixe ensuite sur la β2'-glycoprotéine I un composé viral, contenu dans un fluide biologique ou extrait d'un tissu biologique, de façon à séparer ledit composé viral des composés qui ne sont pas susceptibles de se fixer sur la β2'-glycoprotéine I, et on détecte et/ou on dose le composé viral fixé sur la β2'-glycoprotéine I par tout procédé de détection et/ou de dosage de ce composé viral.

Selon un second mode de mise en oeuvre du procédé, on fixe le composé viral sur un support et on le révèle avec la β2'-glycoprotéine I conjuguée à un marqueur. Le composé viral peut être fixé, soit directement sur le support, soit indirectement, par exemple par l'intermédiaire d'un anticorps. Le marqueur peut, avantageusement, être une enzyme ou un produit radioactif.

La β2'-glycoprotéine I est la glycoprotéine isolée à partir du résidu fixé sur la (les) colonne(s) de chromatographie d'affinité utilisée(s) dans le procédé de purification de l'albumine du plasma sanguin décrit dans FR-A 2 690 444. Selon ce document, on sépare l'albumine des autres protéines par un procédé chromatographique en phase liquide, dans lequel on fait passer la solution aqueuse contenant l'albumine dans au moins une colonne de chromatographie dite "hydrophobe" remplie d'un matériau particulaire apte à retenir une partie des protéines autres que l'albumine ; pour compléter la séparation, on propose également de faire passer la solution aqueuse d'albumine dans au moins une colonne de chromatographie d'affinité contenant un support particulaire neutre ou voisin de la neutralité chargé d'un composé polysulfaté. L'effluent obtenu par ce procédé est constitué par une solution d'albumine purifiée, la majeure partie des protéines autres que l'albumine étant fixée, soit sur la (les) colonne(s) de chromatographie hydrophobe, soit sur la (les) colonne(s) de chromatographie d'affinité.

En soumettant le support de la (des) colonne(s) de chromatographie d'affinité précitée(s) à une élution, de préférence à une élution par augmentation de la force ionique, on obtient une composition protéinique dont le contenu protéinique comporte de 5 à 100 % en poids de β2'-glycoprotéine I.

La β2'-glycoprotéine I a un poids moléculaire de 50 000 ± 3 000 daltons, les 20 premiers acides aminés de la région N-terminale de la β2'-glycoprotéine I sont les suivants : Gly-Arg-Thr-Cys-Pro-Lys-Pro-Asp-Asp-Leu-Pro-Phe-Ser-Thr-Val-Val-Pro-Leu-Lys-Thr et la séquence des acides aminés 315 à 321 est Phe-Trp-Lys-Ser-Asp-Ala-Ser.

Les composés viraux susceptibles d'être reconnus par la β2'-glycoprotéine I peuvent plus particulièrement provenir des virus suivants : de préférence virus de l'hépatite virale B (HBV), virus de l'immunodéficience humaine (HIV₁ et HIV₂), également virus de l'immunodéficience du singe (SIV) ou virus de l'herpès simplex (HSV) ou peuvent être des particules ou des protéines d'origine virale, ou apparentées, présentes dans certaines leucémies ou myélomes ou dans le syndrome de Gougerot-Sjögren.

Selon le premier mode de mise en oeuvre du procédé selon l'invention, on fixe d'abord la β2'-glycoprotéine I sur un support. Il faut noter que lorsque l'on utilise une composition protéinique contenant la β2'-glycoprotéine I et d'autres glycoprotéines, ces autres glycoprotéines peuvent également se fixer sur le support. On utilise, de préférence, un support solide, plus particulièrement constitué par une microplaque de titration, par exemple une microplaque ELISA.

La fixation de la β2'-glycoprotéine I (et éventuellement d'autres glycoprotéines) sur le support se fait par réaction de groupes réactifs de la glycoprotéine avec des sites réactifs du support. Cette réaction est, de préférence, effectuée à une température de 0 à 40°C, la β2'-glycoprotéine I étant, de préférence, mise dans un tampon ayant un pH compris entre 4,5 et 10,5, de préférence entre 5,5 et 6,5. Le tampon peut être du type phosphate ou acétate. La solution obtenue a avantageusement une concentration comprise entre 0,01 à 100 mg/l de β2'-glycoprotéine I. Le support est avantageusement maintenu en contact avec le tampon contenant la β2'-glycoprotéine I à une température de 0 à 40°C et pendant un temps d'incubation compris entre 30 minutes et 24 heures.

Après incubation, on sépare du support le tampon contenant la β2'-glycoprotéine I (et éventuellement les autres glycoprotéines) n'ayant pas réagi et on effectue un lavage du support, de préférence, avec le même tampon que celui qui contenait la β2'-glycoprotéine I. Il peut être nécessaire de saturer les sites actifs du support qui n'ont pas réagi avec la β2'-glycoprotéine I (ou d'autres glycoprotéines). Dans ce cas, on fait réagir sur ces sites actifs d'autres groupes actifs. On utilise avantageusement, dans ce but, une solution d'albumine sérique bovine ou de caséine, en particulier une solution à 2 % dans le tampon utilisé pour la β2'-glycoprotéine I. Après réaction, le support est, également, de préférence, rincé et séché.

Le support rincé et séché sur lequel est fixée au moins la β2'-glycoprotéine peut être stocké avant la fixation des composés viraux. Ce stockage est, de préférence, effectué à +4°C ou -20°C.

Le support sur lequel est fixée au moins la β2'-glycoprotéine I (et éventuellement d'autres glycoprotéines) est ensuite mis en contact avec un fluide biologique susceptible de contenir des composés viraux. Ce fluide est, soit préparé à partir d'organes ou de liquides biologiques d'un malade atteint d'une pathologie virale, soit, provient de surnageants de cultures virales "in vitro". Le liquide biologique peut être un sérum, de l'urine, du liquide céphalorachidien, du liquide synovial, du liquide péritonéal, du liquide pleural, du liquide séminal ou du liquide ascitique. On utilise, de préférence, un plasma sanguin ou sérum. On dilue, de préférence, le fluide biologique à l'aide d'un tampon donnant un pH compris entre 4,5 et 10, avantageusement 5,6. La réaction est, de préférence, effectuée à une température de 0°C à 42°C, avantageusement voisine de 37°C, pendant une période de 30 minutes à 24 heures. On sépare ensuite le fluide biologique et le support portant la β2'-glycoprotéine I (et éventuellement d'autres glycoprotéines), qui a éventuellement fixé un composé viral . On effectue éventuellement ensuite un lavage avec une solution, tamponnée de préférence.

La détection et/ou le dosage du composé viral fixé sur la β2'-glycoprotéine I peuvent se faire par tout moyen connu, tel l'infectiosité ou la détection d'acides nucléiques spécifiques par la technique dite "Polymerase Chain Reaction (PCR)". Cette technique est, par exemple. décrite dans l'article de MULLIS K.B. et FALOONA F.A. dans Methods Enzymol. 1987 155 pages 335-350. La détection et/ou le dosage se font, de préférence, à l'aide d'un anticorps reconnaissant spécifiquement des protéines des composés viraux à détecter. De façon connue, cet anticorps peut être conjugué à un marqueur enzymatique, par exemple de la peroxydase. L'excès d'anticorps est éliminé par lavage. On ajoute ensuite, de façon connue, un substrat spécifique de l'enzyme conjuguée à l'anticorps, substrat qui se transforme, dans des conditions fixées, en un produit coloré. La formation dudit composé coloré indique la présence du composé viral recherché et permet un dosage de ce composé viral.

Selon le second mode de mise en oeuvre du procédé selon l'invention, on fixe le composé viral sur un support solide qui peut être une membrane, par exemple de nitrocellulose ou une microplaque de titration, par exemple une microplaque ELISA.

La fixation du composé viral sur le support se fait par réaction de groupes réactifs du composé viral sur les sites réactifs du support lorsque la fixation est directe, ou par fixation d'un composé, par exemple un anticorps, sur les sites réactifs du support et fixation du composé viral sur ledit composé fixé sur le support. Cette réaction est, de préférence, effectuée à une température de 0 à 40°C, le composé viral étant de préférence mis dans un tampon ayant un pH compris entre 4,5 et 10,5, de préférence entre 6,5 et 7,5. Le tampon peut être du type phosphate ou acétate. Le support est avantageusement maintenu en contact avec le tampon contenant le composé viral à une température de 0 à 40°C et pendant un temps d'incubation compris entre 30 minutes et 24 heures.

Après incubation, on sépare du support le tampon contenant le composé viral n'ayant pas réagi et on effectue un lavage du support, de préférence avec le même tampon que celui qui contenait le composé viral. Il peut être nécessaire de saturer les sites actifs du support qui n'ont pas réagi avec le composé viral. Dans ce cas, on fait réagir sur ces sites actifs d'autres groupes actifs. On utilise avantageusement, dans ce but, une solution d'albumine sérique bovine ou de caséine. Après réaction, le support est également, de préférence, rincé et séché.

Le support sur lequel est fixé le composé viral est ensuite mis en contact avec une solution de β2'-glycoprotéine I conjuguée à un marqueur. On dilue, de préférence, la solution contenant la β2'-glycoprotéine I à l'aide d'un tampon donnant un pH compris entre 4,5 et 10, avantageusement 5,6. La réaction est, de préférence, effectuée à une température de 0°C à 42°C, avantageusement voisine de 37°C pendant une période 30 minutes à 24 heures. On sépare ensuite la solution contenant la β2'-glycoprotéine I qui n'a pas réagi du support portant le composé viral qui a éventuellement fixé la β2'-glycoprotéine I. On effectue éventuellement ensuite un lavage avec une solution, tamponnée de préférence.

La détection et/ou le dosage du composé viral reconnu par la β2'-glycoprotéine I est effectué en ajoutant un substrat spécifique du marqueur conjugué à la β2'-glycoprotéine I lorsque le marqueur est une enzyme ou par mesure de la radioactivité lorsque le marqueur est radioactif.

Les exemples ci-après, donnés à titre purement illustratif et non limitatif, permettront de mieux comprendre l'invention.

### EXEMPLE 1 Détection du virus de l'hépatite B

### A) Fixation de la β2'-glycoprotéine I sur un support

Le support utilisé est une plaque de microtitration de type micro-ELISA à 96 puits à fond plat, commercialisée par la société "DYNATECH". On prépare une solution de β2'-glycoprotéine I ayant une concentration de 10 microgrammes/ml (µg/ml) dans un tampon acétate contenant 0,05 mole/l d'acide acétique et d'acétate et ayant un pH de 5,6 ± 0,05.

On dépose 100 µl de cette solution au fond de chaque puits de la microplaque. Celle-ci est ensuite incubée à +4°C pendant 18 heures. Le liquide de chaque puits est ensuite aspiré. On introduit ensuite dans chaque puits 300 à 400 µl de tampon phosphate contenant 0,01 mole/l de phosphates monosodique et disodique et 0,15 mole/l de chlorure de sodium et ayant un pH de 7,00 ± 0,05. On laisse en contact avec le support pendant 3 minutes et on aspire le tampon ; cette opération de lavage est effectuée 3 fois.

Pour saturer les sites de la microplaque éventuellement encore actifs, on dispose dans chaque puits 200 µl d'une solution d'albumine bovine à 2 % en poids dans le tampon phosphate décrit ci-dessus. La microplaque est ensuite incubée à 37°C pendant 90 minutes. La solution contenue dans chaque puits est ensuite aspirée. On effectue ensuite un lavage en introduisant 300 à 400 µl de tampon phosphate par puits, puis en laissant en contact la solution pendant 3 minutes, et pour finir, en aspirant la solution ; cette opération de lavage est répétée 5 fois. On fait ensuite sécher la plaque.

La plaque sur laquelle est fixée la β2'-glycoprotéine I est ensuite stockée à +4°C ou à -20°C.

### B) Fixation de composés viraux

On a utilisé 48 échantillons de sérum sanguin de malades atteints d'hépatite virale B contenant l'antigène du virus HBV (AgHB_{S}) et à titre comparatif 48 échantillons de sérum sanguin de donneurs sains. Chaque échantillon sérique est dilué 100 fois. La dilution est effectuée à l'aide d'un tampon (acide acétique/acétate de sodium) ayant une concentration en acétate et acide acétique de 0,05 mole/l et un pH de 5,6 ± 0,05. On dépose 100 µl de solution au fond de chaque puits de la plaque préparée et stockée selon A. La plaque est incubée à +37°C pendant une période de 90 minutes. Après cette incubation, on effectue un lavage en introduisant 300 µl de tampon phosphate dans chaque puits, on laisse en contact pendant 2 minutes et on aspire la solution de tampon ; on renouvelle cette opération de lavage 3 fois.

### C) Détection de composés viraux

On ajoute par puits 100 µl d'une solution d'anticorps monoclonal spécifique contre AgHB_{S} du virus de l'hépatite B conjugué à la péroxydase. On laisse la plaque incuber à 37°C pendant 60 minutes. A la suite de cette incubation, le contenu des puits de la plaque est aspiré. On introduit 300 µl de tampon phosphate dans chaque puits et après un temps de contact de 2 minutes, on aspire le tampon : cette opération de lavage est renouvelée 3 fois.

On ajoute par puits 100 µl d'une solution d'o-phénylène-diamine, 2HCl dans un tampon de citrate de sodium. On laisse incuber pendant 30 minutes à température ambiante, puis on arrête la réaction en rajoutant à chaque puits 50 µl de H₂SO₄,2N. On mesure l'absorbance à 492 nm obtenue en fin de réaction à l'aide d'un robot lecteur de plaque.

La moyenne des absorbances obtenue pour chaque malade ou donneur sont données dans le tableau I en UDO x 100. Les échantillons A1 à D12 correspondent aux 48 sérums de malades et les échantillons E1 à H12 correspondent aux sérums des donneurs.

Ces résultats montrent que par le test selon la présente demande :
1) tous les sérums de donneurs sains ont effectivement été reconnus comme négatifs,
2) 77,1 % des sérums de malades ont été reconnus positifs et seulement 22,9 % comme négatifs.

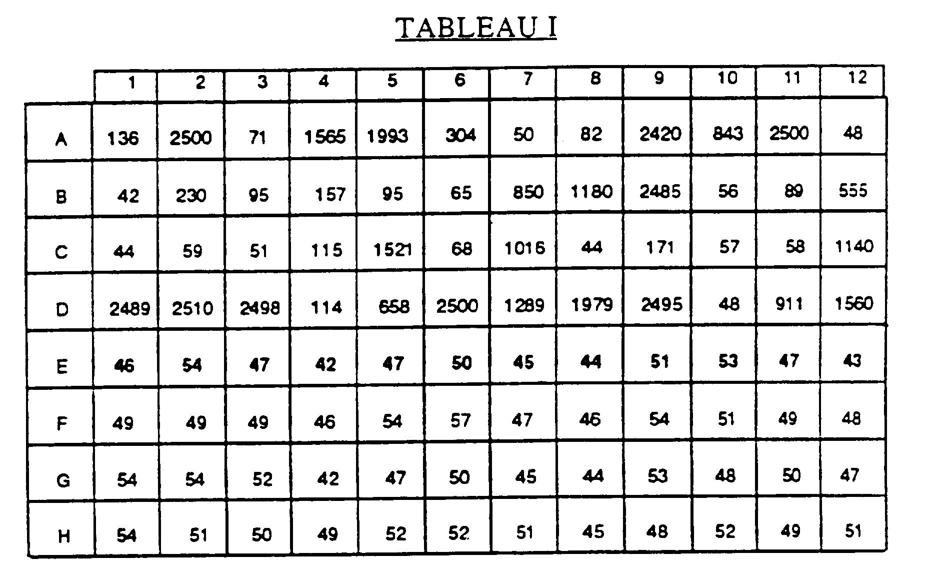

### EXEMPLE 2 Détection du virus HIV₁

Le support utilisé est une plaque de microtitration de type micro-ELISA à 96 puits et à fond plat, commercialisée par la société "DYNATECH". On prépare une solution de β2'-glycoprotéine I ayant une concentration de 10 µg/mB dans un tampon acétate contenant 0,05 mole/l d'acide acétique et d'acétate de sodium et ayant un pH de 5,6 ± 0,05. On dépose 100 µl de cette solution au fond de chaque puits de la microplaque. Celle-ci est ensuite incubée à +4°C pendant 18 heures. Le liquide de chaque puits est ensuite aspiré. On introduit ensuite dans chaque puits 300 à 400 µl de tampon phosphate contenant 0,01 mole/l de phosphates monosodique et disodique et 0,15 mole/l de chlorure de sodium et ayant un pH de 7,00 ± 0,05. On laisse en contact avec le support pendant 3 minutes et on aspire le tampon ; cette opération de lavage est effectuée 3 fois.

Pour saturer les sites de la microplaque éventuellement encore actifs, on dispose dans chaque puits 200 µl d'une solution d'albumine bovine à 2 % en poids dans le tampon phosphate décrit ci-dessus. La microplaque est ensuite incubée à 37°C pendant 90 minutes. La solution contenue dans chaque puits est ensuite aspirée. On effectue ensuite un lavage en introduisant 300 à 400 µl de tampon phosphate par puits, puis en laissant en contact la solution pendant 3 minutes, et pour finir en aspirant la solution ; cette opération de lavage est répétée 5 fois. On fait ensuite sécher la plaque.

On a utilisé 17 échantillons de sérum de donneurs sains et 17 échantillons de sérum de malades infectés par le HIV₁. L'échantillon sérique est dilué 100 fois dans le tampon acétate décrit ci-dessus. On dépose 100 µl de solution au fond de chaque puits de la plaque préparée comme ci-dessus. La plaque est incubée à +37°C pendant une période de 90 minutes. Après cette incubation, on effectue un lavage en introduisant 300 µl de tampon phosphate dans chaque puits, on laisse en contact pendant 2 minutes et on aspire la solution de tampon ; on renouvelle cette opération de lavage 5 fois.

On ajoute, ensuite, par puits 100 µl d'une solution d'anticorps polyclonaux humains spécifiques du HIV₁, conjugués à la péroxydase, commercialisés par l'Institut Pasteur-SANOFI. On laisse la plaque incuber à +37°C pendant 60 minutes. A la suite de cette incubation, le contenu des puits de la plaque est aspiré. On introduit 300 µl de tampon phosphate dans chaque puits et après un temps de contact de 2 minutes, on aspire le tampon : cette opération de lavage est renouvelée 3 fois.

On ajoute par puits 100 µl d'une solution d'o-phénylène-diamine, 2 HCI dans un tampon citrate de sodium. On laisse incuber pendant 30 minutes à température ambiante, puis on arrête la réaction en rajoutant à chaque puits 50 µl de H₂SO₄,2N. On mesure en UDO l'absorbance à 492 nm obtenue en fin de réaction à l'aide d'un robot lecteur de plaque.

Les résultats obtenus sont donnés dans le tableau II ci-dessous :

| Echantillons | Donneurs sains | Malades |
|---|---|---|
| 1 | 0,088 | 0,549 |
| 2 | 0,058 | 0,544 |
| 3 | 0,074 | 2,151 |
| 4 | 0,070 | 0,892 |
| 5 | 0,071 | 2,900 |
| 6 | 0,080 | 0,121 |
| 7 | 0,070 | 0,160 |
| 8 | 0,072 | 0,134 |
| 9 | 0,081 | 0,233 |
| 10 | 0,078 | 1,330 |
| 11 | 0,070 | 0,141 |
| 12 | 0,073 | 0,093 |
| 13 | 0,075 | 0,291 |
| 14 | 0,076 | 0,644 |
| 15 | 0,082 | 0,350 |
| 16 | 0,080 | 0,550 |
| 17 | 0,083 | 0,510 |

On voit que
- pour tous les sérums de donneurs sains, l'absorbance est inférieure à 0,1;
- parmi les sérums de malades,
   - 12 ont une absorbance supérieure à 0,2 UDO (soit 70 %)
   - 4 ont une absorbance comprise entre 0,1 et 0,2 UDO (soit 23,5 %)
   - (par conséquent 93,5 % peuvent être considérés comme positifs)
   - 1 est considéré comme négatif (6,5 %) ayant une absorbance inférieure à 0,1.

### EXEMPLE 3

On filtre, à travers une membrane de nitrocellulose (BA 83, Schleicher et Schuell), par aspiration en différentes zones de quelques mm³ (1 à 3) différentes quantités 300 ng, 30 ng, 3 ng et 0 ng de protéine recombinante p26-HIV₂ ROD en solution dans un mélange aqueux d'éthanolamine (12 g/l) et de glycine (24 g/l), de façon à retenir sur la membrane au moins une partie de la protéine recombinante. On sature ensuite la membrane par une incubation en présence de 1 % en poids de polyvinylpyrrolidone ayant un poids moléculaire voisin de 10 000 kdaltons en solution dans le même mélange d'éthanolamine et de glycine que ci-dessus. On rince ensuite avec une solution de tampon contenant 5 mMoles de NaH₂PO₄, 5 mM de Na₂HPO₄, 125 mM de NaCl et 0,05 % en poids de "TWEEN 20".

On fait réagir sur la membrane environ 40 ng de β2'-glycoprotéine I couplée à de la phosphatase alcaline en solution dans 200 µl du tampon phosphaté décrit ci-dessus pendant 1 heure à 37°C. On rince ensuite 3 fois avec la solution d'éthanolamine et de glycine ci-dessus. On révèle l'activité de phosphatase alcaline en présence de nitro-blue-tétrazolium (NBT) et 4-bromo 4-chloro 3-indolyl phosphate (BCIP) dans une solution à 50 mM de tris(hydroxyméthyl)aminométhane à pH 8 et à 0,1 M de NaCl. On voit sur la membrane que la β2'-glycoprotéine I permet de révéler la présence de la protéine recombinée p26-HIV₂ sur les zones où elle est présente, la membrane étant plus ou moins colorée dans ces zones. La zone de contrôle n'est pas modifiée.

Dans les mêmes conditions, il n'y a pas de réaction avec la phosphatase alcaline seule.

### EXEMPLE 4

Dans des conditions identiques à celles de l'exemple 3, sauf que l'incubation a été faite en présence de sérum de veau foetal dilué 2 000 fois au lieu de polyvinylpyrrolidone, on a observé une réaction avec la protéine recombinante gp 160-HIV₁,LAI sur les zones de la membrane imprégnées de protéine recombinante.

## Revendications

1. Procédé de détection et/ou de dosage de composés viraux, caractérisé par le fait qu'on fixe les composés viraux à l'aide de la β2'-glycoprotéine I, qui est susceptible d'être obtenue dans un traitement de purification de l'albumine du plasma sanguin par un procédé de chromatographie en phase liquide dans lequel on fait passer la solution aqueuse contenant l'albumine dans au moins une colonne de chromatographie hydrophobe remplie d'un matériau particulaire apte à retenir une partie des protéines autres que l'albumine, puis on fait passer la solution d'albumine dans au moins une colonne de chromatographie d'affinité contenant un support particulaire neutre ou voisin de la neutralité chargé d'un composé polysulfaté et on soumet le support de la (des) colonne(s) de chromatographie d'affinité précitée à une élution de façon à obtenir une composition protéinique contenant la β2'-glycoprotéine I, dont les 20 premiers acides aminés de la région N-terminale sont Gly-Arg-Thr-Cys-Pro-Lys-Pro-Asp-Asp-Leu-Pro-Phe-Ser-Thr-Val-Val-Pro-Leu-Lys-Thr et dont le poids moléculaire est 50 000 ± 3 000 daltons.

2. Procédé selon la revendication 1, caractérisé par le fait qu'on utilise la β2'-glycoprotéine I sous forme pure ou sous forme de composition protéinique en mélange avec d'autres glycoprotéines.

3. Procédé selon l'une des revendications 1 ou 2, caractérisé par le fait qu'on fixe sur la β2'-glycoprotéine I un composé viral contenu dans un fluide biologique ou extrait d'un tissu biologique de façon à séparer ledit composé viral des composés qui ne sont pas susceptibles de se fixer sur la β2'-glycoprotéine I et que l'on détecte et/ou dose le composé viral fixé sur la β2'-elycoprotéine I par tout procédé connu de détection de ce composé viral.

4. Procédé selon l'une des revendications 1 ou 2, caractérisé par le fait qu'on fixe le composé viral sur un support et on le révèle avec la β2'-glycoprotéine I conjuguée à un marqueur.

5. Procédé selon l'une des revendications 1 à 4, caractérisé par le fait que les composés viraux fixés par la β2'-glycoprotéine I proviennent du virus de l'hépatite virale B (HBV) et du virus de l'immunodéficience humaine (HIV₁ et HIV₂).

6. Procédé selon l'une des revendications 1 à 4, caractérisé par le fait que les composés viraux fixés par la β2'-glycoprotéine I proviennent du virus de l'immunodéficience du singe (SIV) ou du virus de l'herpès simplex (HSV)

7. Procédé selon l'une des revendications 1 à 4, caractérisé par le fait que les composés viraux fixés par la β2'-glycoprotéine I sont des particules, ou des protéines d'origine virale, ou apparentées, présentes dans des leucémies ou myélomes ou dans le syndrome de Gougerot-Sjögren.

8. Procédé selon l'une des revendications 3 à 7, caractérisé par le fait que le support sur lequel on fixe la β2'-glycoprotéine I ou le composé viral est un support solide.

9. Procédé selon la revendication 8, caractérisé par le fait que le support solide est une microplaque ELISA.

10. Procédé selon les revendications 3, 5 à 9, caractérisé par le fait qu'avant la réaction de fixation de la β2'-glycoprotéine I sur le support, la β2'-glycoprotéine I est mise en solution dans un tampon ayant un pH compris entre 4,5 et 10,5.

11. Procédé selon la revendication 10, caractérisé par le fait que le tampon est du type phosphate ou acétate.

12. Procédé selon l'une des revendications 10 ou 11, caractérisé par le fait que la β2'-glycoprotéine I est à une concentration comprise entre 0,01 et 100 mg/l dans le tampon.

13. Procédé selon l'une des revendications 10 à 12, caractérisé par le fait que l'on réalise la réaction de fixation de la β2'-glycoprotéine I sur le support en mettant le tampon contenant la β2'-glycoprotéine I en contact avec le support à une température de 0 à 40°C pendant un temps d'incubation compris entre 30 minutes et 24 heures.

14. Procédé selon l'une des revendications 10 à 13, caractérisé par le fait que l'on sépare du support la quantité de solution contenant la β2'-glycoprotéine I, qui n'a pas réagi avec ledit support, et on lave le support.

15. Procédé selon la revendication 14, caractérisé par le fait qu'on lave le support avec le même tampon que celui utilisé pour mettre en solution la β2'-glycoprotéine I.

16. Procédé selon l'une des revendications 1 à 3, 5 à 15, caractérisé par le fait qu'après la fixation de la β2'-glycoprotéine I, on sature les sites actifs du support qui n'ont pas réagi avec la β2'-glycoprotéine I.

17. Procédé selon la revendication 16, caractérisé par le fait qu'on sature les sites à l'aide d'une solution d'albumine sérique bovine, ou de caséine.

18. Procédé selon l'une des revendications 4, 5 à 17, caractérisé par le fait que le support sur lequel est fixée au moins la β2'-glycoprotéine I est stocké avant la fixation des composés viraux.

19. Procédé selon l'une des revendications 3, 5 à 18, caractérisé par le fait que le fluide biologique est un sérum, un plasma, de l'urine, du liquide céphalorachidien, du liquide synovial, du liquide péritonéal, du liquide pleural, du liquide séminal, du liquide ascitique ou du surnageant de culture virale "in vitro" ou un extrait cellulaire.

20. Procédé selon l'une des revendications 3, 5 à 19, caractérisé par le fait que le fluide biologique est dilué à l'aide d'un tampon donnant un pH compris entre 4,5 et 10,0.

21. Procédé selon l'une des revendications 3, 5 à 20, caractérisé par le fait que la réaction de fixation virale est effectuée à une température comprise entre 0 et 42°C, pendant une période de 30 minutes à 24 heures.

22. Procédé selon la revendication 21, caractérisé par le fait qu'après la fixation virale, on sépare du support le fluide biologique et on lave ledit support.

23. Procédé selon l'une des revendications 3, 5 à 22, caractérisé par le fait qu'on réalise, à l'aide d'un anticorps, la détection et/ou le dosage du composé viral fixé sur le support par l'intermédiaire de la β2'-glycoprotéine I.

24. Procédé selon la revendication 23, caractérisé par le fait que l'anticorps est conjugué à un marqueur enzymatique.

25. Procédé selon la revendication 24, caractérisé par le fait qu'on ajoute un substrat spécifique de l'enzyme conjuguée à l'anticorps.

26. Support sur lequel est fixée au moins la β2'-glycoprotéine I préparé en vue de la mise en oeuvre du procédé selon la revendication 18.

## Patentansprüche

1. Verfahren zum Nachweis und/oder zur Bestimmung viraler Substanzen, dadurch gekennzeichnet, daß man die viralen Substanzen mit Hilfe des β2'-Glycoproteins I bindet, welches bei einer Aufbereitung zur Reinigung von Blutplasmaalbumin über ein Flüssigkeitsphasenchromatographie-Verfahren erhältlich ist, bei dem man die Albumin enthaltende wäßrige Lösung durch wenigstens eine Hydrophobchromatographiesäule schickt, die mit einem teilchenförmigen Material gefüllt ist, welches geeignet ist, einen Teil der von Albumin verschiedenen Proteine zurückzuhalten, dann die Albuminlösung durch wenigstens eine Affinitätschromatographiesäule schickt, die einen teilchenförmigen neutralen oder nahezu neutralen, mit einer polysulfatierten Substanz beladenen Träger enthält, und man den Träger der vorbezeichneten Affinitätschromatographiesäule(n) einer Elution unterzieht, so daß eine Proteinzusammensetzung erhalten wird, die das β2'-Glycoprotein I enthält, dessen 20 erste Aminosäuren der N-terminalen Region Gly-Arg-Thr-Cys-Pro-Lys-Pro-Asp-Asp-Leu-Pro-Phe-Ser-Thr-Val-val-Pro-Leu-Lys-Thr sind und dessen Molekulargewicht 50000 ± 3000 Dalton beträgt.

2. Verfahren nach Anspruch 1, dadurch gekennzeichnet, daß man das β2'-Glycoprotein I in reiner Form oder in Form einer Proteinzusammensetzung im Gemisch mit anderen Glycoproteinen verwendet.

3. Verfahren nach einem der Ansprüche 1 oder 2, dadurch gekennzeichnet, daß man eine in einer biologischen Flüssigkeit oder einem Extrakt eines biologischen Gewebes enthaltene virale Substanz an das β2'-Glycoprotein I bindet, so daß man diese virale Substanz von Substanzen abtrennt, die nicht an das β2'-Glycoprotein I binden können, und man die an das β2'-Glycoprotein I gebundene virale Substanz durch ein für den Nachweis dieser viralen Substanz bekanntes Verfahren nachweist und/oder bestimmt.

4. Verfahren nach einem der Ansprüche 1 oder 2, dadurch gekennzeichnet, daß man die virale Substanz an einen Träger bindet und sie mit dem an einen Marker gekoppelten β2'-Glycoprotein I anzeigt.

5. Verfahren nach einem der Ansprüche 1 bis 4, dadurch gekennzeichnet, daß die durch das β2'-Glycoprotein I gebundenen viralen Substanzen vom Hepatitis B-Virus (HBV) und vom humanen Immundefekt-Virus (HIV₁ und HIV₂) herrühren.

6. Verfahren nach einem der Ansprüche 1 bis 4, dadurch gekennzeichnet, daß die durch das β2'-Glycoprotein I gebundenen viralen Substanzen vom Affen-Immundefekt-Virus (SIV) oder vom Herpes simplex-Virus (HSV) herrühren.

7. Verfahren nach einem der Ansprüche 1 bis 4, dadurch gekennzeichnet, daß die durch das β2'-Glycoprotein I gebundenen viralen Substanzen Teilchen oder Proteine viralen Ursprungs oder verwandte Teilchen oder Proteine sind, die bei Leukämien oder Myelomen oder bei dem Gougerot-Sjögren-Syndrom auftreten.

8. Verfahren nach einem der Ansprüche 3 bis 7, dadurch gekennzeichnet, daß der Träger, an den nan das β2'-Glycoprotein I oder die virale Substanz bindet, ein fester Träger ist.

9. Verfahren nach Anspruch 8, dadurch gekennzeichnet, daß der feste Träger eine ELISA-Mikroplatte ist.

10. Verfahren nach einem der Ansprüche 3 oder 5 bis 9, dadurch gekennzeichnet, daß vor der Reaktion zur Bindung des β2'-Glycoproteins I an den Träger das β2'-Glycoprotein I in einem Puffer mit einem pH-Wert von 4,5 bis 10,5 in Lösung gebracht wird.

11. Verfahren nach Anspruch 10, dadurch gekennzeichnet, daß der Puffer vom Phosphat- oder Acetattyp ist.

12. Verfahren nach einem der Ansprüche 10 oder 11, dadurch gekennzeichnet, daß das β2'-Glycoprotein I in dem Puffer in einer Konzentration von 0,01 bis 100 mg/l vorliegt.

13. Verfahren nach einem der Ansprüche 10 bis 12, dadurch gekennzeichnet, *daß* man die Reaktion zur Bindung des β2'-Glycoproteins I an den Träger durchführt, indem man den das β2'-Glycoprotein I enthaltenen Puffer mit dem Träger bei einer Temperatur von 0 bis 40°C während einer Inkubationszeit von 30 Minuten bis 24 Stunden in Xontakt bringt.

14. Verfahren nach einem der Ansprüche 10 bis 13, dadurch gekennzeichnet, daß man von dem Träger die Lösung abtrennt, die das β2'-Glycoprotein I enthält, welches nicht mit dem Träger reagiert hat, und man den Träger wäscht.

15. Verfahren nach Anspruch 14, dadurch gekennzeichnet, daß man den Träger mit dem gleichen Puffer wäscht, den man verwendet hat, um das β2'-Glycoprotein I in Lösung zu bringen.

16. Verfahren nach einem der Ansprüche 1 bis 3 oder 5 bis 15, dadurch gekennzeichnet, daß man nach Bindung des β2'-Glycoproteins I die aktiven Zentren des Trägers, die nicht mit dem β2'-Glycoprotein I reagiert haben, sättigt.

17. Verfahren nach Anspruch 16, dadurch gekennzeichnet, daß man die Zentren mit Hilfe einer Rinderserumalbunin- oder Caseinlösung sättigt.

18. Verfahren nach einem der Ansprüche 4 oder 5 bis 17, dadurch gekennzeichnet, daß der Träger, an dem zumindest das β2'-Glycoprotein I gebunden ist, vor der Bindung viraler Substanzen gelagert wird.

19. Verfahren nach einem der Ansprüche 3 oder 5 bis 18, dadurch gekennzeichnet, daß die biologische Flüssigkeit ein Serum, ein Plasma, Urin, Cephalorachidialflüssigkeit, Synovialflüssigkeit, Peritonealflüssigkeit, Pleuralflüssigkeit, Seminalflüssigkeit, Ascitesflüssigkeit oder der Überstand viraler Kulturen "in vitro" oder ein zellulärer Extrakt ist.

20. Verfahren nach einem der Ansprüche 3 oder 5 bis 19, dadurch gekennzeichnet, daß die biologische Flüssigkeit mit Hilfe eines Puffers verdünnt wird, wobei sich ein pH-Wert von 4,5 bis 10,0 ergibt.

21. Verfahren nach einem der Ansprüche 3 oder 5 bis 20, dadurch gekennzeichnet, daß die Reaktion zur viralen Bindung bei einer Temperatur von 0 bis 42°C während einer Dauer von 30 Minuten bis 24 Stunden durchgeführt wird.

22. Verfahren nach Anspruch 21, dadurch gekennzeichnet, daß man nach der viralen Bindung die biologische Flüssigkeit vom Träger abtrennt und den Träger wäscht.

23. Verfahren nach einem der Ansprüche 3 oder 5 bis 22, dadurch gekennzeichnet, daß man den Nachweis und/oder die Bestimmung der unter Zwischenschaltung des β2'-Glycoproteins I an den Träger gebundenen viralen Substanz mit Hilfe eines Antikörpers vornimmt.

24. Verfahren nach Anspruch 23, dadurch gekennzeichnet, daß der Antikörper an einen enzymatischen Marker gekoppelt ist.

25. Verfahren nach Anspruch 24, dadurch gekennzeichnet, daß man ein Substrat zugibt, für welchen das an den Antikörper - gekoppelte Enzym spezifisch ist.

26. Träger, an den zumindest das im Hinblick auf die Ausführung des Verfahrens gemäß Anspruch 18 hergestellte β2'-Glycoprotein I gebunden ist.

## Claims

1. Method for detecting and/or assaying viral compounds, characterized in that the viral compounds are bound using β2'-glycoprotein I, which can be obtained in a treatment for the purification of blood plasma albumin by a liquid phase chromatographic process in which the aqueous solution containing the albumin is passed through at least one hydrophobic chromatography column filled with a particulate material capable of retaining some of the proteins other than the albumin, and the albumin solution is then passed through at least one affinity chromatography column containing a particulate support which is neutral or close to neutrality, charged with a polysulphate compound, and the support for the abovementioned affinity chromatography column(s) is subjected to elution so as to obtain a protein composition containing the β2'-glycoprotein I, the first 20 amino acids at the N-terminal region of which are Gly-Arg-Thr-Cys-Pro-Lys-Pro-Asp-Asp-Leu-Pro-Phe-Ser-Thr -Val-Val-Pro-Leu-Lys-Thr and the molecular weight of which is 50,000 ± 3000 daltons.

2. Method according to Claim 1, characterized in that β2'-glycoprotein I is used in pure form or in the form of a protein composition mixed with other glycoproteins.

3. Method according to either of Claims 1 and 2, characterized in that a viral compound contained in a biological fluid or extracted from a biological tissue is bound to the β2'-glycoprotein I so as to separate the said viral compound from the compounds which are not capable of binding to β2'-glycoprotein I, and in that the viral compound bound to the β2'-glycoprotein I is detected and/or assayed by any known method for detecting this viral compound.

4. Method according to either of Claims 1 and 2, characterized in that the viral compound is bound to a support and is exposed with β2'-glycoprotein I conjugated with a label.

5. Method according to one of Claims 1 to 4, characterized in that the viral compounds bound by β2'-glycoprotein I originate from hepatitis B virus (HBV) and from human immunodeficiency virus (HIV₁ and HIV₂).

6. Method according to one of Claims 1 to 4, characterized in that the viral compounds bound by β2'-glycoprotein I originate from simian immunodeficiency virus (SIV) or from herpes simplex virus (HSV).

7. Method according to one of Claims 1 to 4, characterized in that the viral compounds bound by β2'-glycoprotein I are particles, or proteins of viral origin, or similar to these, which are present in leukaemias or myelomas or in the Gougerot-Sjögren syndrome.

8. Method according to one of Claims 3 to 7, characterized in that the support on which β2'-glycoprotein I or the viral compound is bound is a solid support.

9. Method according to Claim 8, characterized in that the solid support is an ELISA microplate.

10. Method according to Claims 3 and 5 to 9, characterized in that before the reaction for binding β2'-glycoprotein I to the support, the β2'-glycoprotein I is dissolved in a buffer having a pH between 4.5 and 10.5.

11. Method according to Claim 10, characterized in that the buffer is of the phosphate or acetate type.

12. Method according to either of Claims 10 and 11, characterized in that the β2'-glycoprotein I is at a concentration between 0.01 and 100 mg/l in the buffer.

13. Method according to one of Claims 10 to 12, characterized in that the reaction for binding β2'-glycoprotein I to the support is performed by placing the buffer containing the β2'-glycoprotein I in contact with the support at a temperature of 0 to 40°C for an incubation time of between 30 minutes and 24 hours.

14. Method according to one of Claims 10 to 13, characterized in that the amount of solution containing the β2'-glycoprotein I which has not reacted with the support is separated from the said support and the support is washed.

15. Method according to Claim 14, characterized in that the support is washed with the same buffer as that used to dissolve the β2'-glycoprotein I.

16. Method according to one of Claims 1 to 3 and 5 to 15, characterized in that after binding the 82"-glycoprotein I, the active sites of the support which have not reacted with the β2'-glycoprotein I are saturated.

17. Method according to Claim 16, characterized in that the sites are saturated using a bovine serum albumin solution or casein solution.

18. Method according to one of Claims 4 and 5 to 17, characterized in that the support, on which at least the β2'-glycoprotein I is bound, is stored before the viral compounds are bound.

19. Method according to one of Claims 3 and 5 to 18, characterized in that the biological fluid is a serum, a plasma, urine, cephalorrachidian fluid, synovial fluid, peritoneal fluid, pleural fluid, seminal fluid, ascitic fluid or supernatant from "in vitro" viral culture or a cell extract.

20. Method according to one of Claims 3 and 5 to 19, characterized in that the biological fluid is diluted using a buffer giving a pH between 4.5 and 10.0.

21. Method according to one of Claims 3 and 5 to 20, characterized in that the viral binding reaction is carried out at a temperature between 0 and 42°C, for a period of 30 minutes to 24 hours.

22. Method according to Claim 21, characterized in that after the viral binding, the biological fluid is separated from the support and the said support is washed.

23. Method according to one of Claims 3 and 5 to 22, characterized in that the viral compound bound to the support via β2'-glycoprotein I is detected and/or assayed using an antibody.

24. Method according to Claim 23, characterized in that the antibody is conjugated with an enzymatic label.

25. Method according to Claim 24, characterized in that a substrate which is specific for the enzyme conjugated with the antibody is added.

26. Support on which at least β2'-glycoprotein I, prepared for the purpose of carrying out the method according to Claim 18, is bound.
